(19) Europäisches Patentamt / European Patent Office / Office européen des brevets

(11) **EP 3 009 133 B1**

(12) **EUROPEAN PATENT SPECIFICATION**

(45) Date of publication and mention of the grant of the patent:
**05.10.2016 Bulletin 2016/40**

(51) Int Cl.:
*A61K 31/122* (2006.01)     *A61K 31/4415* (2006.01)
*A61K 31/525* (2006.01)     *A61K 31/714* (2006.01)
*A61K 33/06* (2006.01)     *A61K 33/26* (2006.01)
*A61K 33/30* (2006.01)     *A61P 1/00* (2006.01)

(21) Application number: **15171583.6**

(22) Date of filing: **11.06.2015**

(54) **PHARMACEUTICAL COMPOSITION FOR USE IN THE TREATMENT OR PREVENTION OF VITAMIN AND MINERAL DEFICIENCIES IN PATIENT WHICH HAVE BEEN SUBJECTED TO GASTRIC BYPASS-SURGERY**

PHARMAZEUTISCHE ZUSAMMENSETZUNG ZUR VERWENDUNG BEI DER BEHANDLUNG ODER PRÄVENTION VON VITAMIN- UND MINERALIENMANGEL BEI PATIENTEN, DIE EINER MAGENBYPASS-OPERATION UNTERZOGEN WURDEN

COMPOSITION PHARMACEUTIQUE POUR UNE UTILISATION DANS LE TRAITEMENT OU LA PRÉVENTION DE DÉFICIENCES EN VITAMINES ET EN MINÉRAUX CHEZ DES PATIENTS QUI ONT ÉTÉ SOUMIS À UNE CHIRURGIE DE BYPASS GASTRIQUE.

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **17.10.2014  NL 2013645**

(43) Date of publication of application:
**20.04.2016 Bulletin 2016/16**

(73) Proprietor: **Fit For Me B.V.**
**3021 DM  Rotterdam (NL)**

(72) Inventor: **Hamer, Simon**
**2515 TV Den Haag (NL)**

(74) Representative: **Nederlandsch Octrooibureau**
**P.O. Box 29720**
**2502 LS The Hague (NL)**

(56) References cited:
**US-A1- 2010 227 001**

- **GASTEYGER,: "Nutritional deficiencies after Roux-en-Y gastric bypass for morbid obesity often cannot be prevented by standard multivitamin supplementation", AMERICAN JOURNAL OF CLINICAL NUTRITION, vol. 87, no. 5, May 2008 (2008-05), pages 1128-1133, XP055178436, ISSN: 0002-9165**

- **ANGEL G. VARGAS-RUIZ ET AL: "Prevalence of Iron, Folate, and Vitamin B12 Deficiency Anemia After Laparoscopic Roux-en-Y Gastric Bypass", OBESITY SURGERY, vol. 18, no. 3, 23 January 2008 (2008-01-23), pages 288-293, XP055178452, ISSN: 0960-8923, DOI: 10.1007/s11695-007-9310-0**

- **RADMILA LEVINSON ET AL: "Pharmacotherapy Prevention and Management of Nutritional Deficiencies Post Roux-en-Y Gastric Bypass", OBESITY SURGERY, vol. 23, no. 7, 5 April 2013 (2013-04-05), pages 992-1000, XP055178461, ISSN: 0960-8923, DOI: 10.1007/s11695-013-0922-2**

- **POITOU BERNERT ET AL: "Nutritional deficiency after gastric bypass: diagnosis, prevention and treatment", DIABETES & METABOLISM, PARIS, AMSTERDAM, NL, vol. 33, no. 1, 7 February 2007 (2007-02-07), pages 13-24, XP005943034, ISSN: 1262-3636, DOI: 10.1016/J.DIABET.2006.11.004**

- **LEBOWITZ D ET AL: "Nutritional deficiency after gastric bypass surgery: A review of the literature and guidelines for follow-up", REVUE MEDICALE SUISSE 20120321 EDITIONS MEDECINE ET HYGIENE CHE, vol. 8, no. 333, 21 March 2012 (2012-03-21), pages 649-654, XP008175623, ISSN: 1660-9379**

EP 3 009 133 B1

- **LEDOUX S ET AL: "Long-term evolution of nutritional deficiencies after gastric bypass: An assessment according to compliance to medical care", ANNALS OF SURGERY, J.B. LIPPINCOTT COMPANY, PHILADELPHIA, US, vol. 259, no. 6, June 2014 (2014-06), pages 1104-1110, XP008175622, ISSN: 0003-4932, DOI: 10.1097/SLA.0000000000000249**

**Description**

TECHNICAL FIELD OF THE INVENTION

The present invention relates to a pharmaceutical composition and to a therapeutic combination for use in the treatment or prevention of vitamin and mineral deficiencies in patients which have been subjected to gastric bypass-surgery. The present invention further relates to a pharmaceutical composition as such.

BACKGROUND OF THE INVENTION

Bariatric surgery was first introduced in the 1960's in the United States as a treatment for morbid obesity. In the last decade the number of bariatric procedures has grown rapidly. This increase is largely due to the increase in the number of people suffering from morbid obesity. Patients suffering from morbid obesity have an increased risk for many different diseases, such as type II diabetes, coronary disease, hyperlipidemia, hypertension, sleep apnea, osteoarthritis, several types of cancer and liver steatosis. Furthermore, morbid obesity is also associated, as its name already suggests, with a shortened life expectancy and a reduced quality of life.

Dietary advice, encouraging patients to do more physical exercise and drug therapy are often not effective enough in the treatment of morbid obesity. Hence, surgical procedures are now commonly used to treat morbid obesity. In general bariatric surgery can be divided in restrictive techniques and in a combination of restrictive and malabsorptive techniques. The gastric band and the gastric sleeve are examples of restrictive bariatric surgery. They provide an average excess weight loss of 30-50%. The Roux-en-Y gastric bypass (hereinafter: RYGB), the duodenal switch and the Scopinaro are examples of combined techniques. The RYGB is the most commonly used technique. Although this technique has a higher risk for complications, it gives a greater excess weight loss, i.e. between 50 to 90%.

In general, the relatively lighter patients are often treated by applying a gastric band or by using a gastric sleeve. The relatively heavier group of patients is most often treated by using a RYGB.

The RYGB is in general performed as follows. Using a stapling device the stomach is cut into two parts. A very small part of the stomach (referred to as pouch) of about 30-45cc remains. Subsequently the small intestine is located and measured about 50 centimeters from the Treitz ligament and the intestine is placed towards the pouch, and after making a passage it is attached to the pouch. There is a feedback loop to the pouch, where one end is detached. This loose end has now become the drain of gastric juice, bile and digestive enzymes from the pancreas. What results is a pouch, allowing patients to eat less. Also food now passes through the first two meters of intestines without digestive juices coming into contact with it. The remaining 2 to 4 meters of small intestine remain for the absorption of food, which is a reduction of 33-50% of the total small intestine surface.

Due to the limitation of food intake, the patients which have been subjected to a RYGB find it difficult to keep nutrients levels such as vitamins and minerals at the same level as before said procedure was carried out. Furthermore, many patients which have had a RYGB have difficulties eating certain foods containing essential nutrients. Clinical studies have shown that almost two thirds of all RYGB patients avoid certain foods as they may cause these patients to vomit. Without wishing to be bound by any theory, it is assumed that this is mainly due to a combination of insufficient chewing of the food and the slow passage of certain food product through the (reduced) stomach. Furthermore, due to removal of a significant part of the stomach only small amounts of gastric acid and intrinsic factor is produced. Since gastric acid is essential for the uptake of iron from food and intrinsic factor is essential for absorbtion of vitamin B12 the levels of these nutrients is low in patients which have been subjected to a RYGB.

In order to balance the reduced intake and absorbtion of these nutrients, patients which have been subjected to a RYGB are prescribed multivitamin preparations comprising the recommended daily dose of these nutrients for health subjects. However, clinical studies have now shown that the use of these multivitamin preparations does not restore the essential vitamin and mineral balance in patients which have been subjected to gastric bypass-surgery, in particular patients which have been subjected to a Roux-en-Y gastric bypass procedure.

Hence, a need remains for a medicament which is able to better restore the vitamin and mineral balance in patients which have been subjected to gastric bypass-surgery, in particular to a Roux-en-Y gastric bypass.

SUMMARY OF THE INVENTION

A first aspect of the present invention relates to a pharmaceutical composition for use in the treatment or prevention of vitamin and mineral deficiencies in patients which have been subjected to gastric bypass-surgery comprising:

- vitamin B 12 or a source thereof;
- vitamin B6 or a source thereof;

- iron or a source thereof;
- a pharmaceutically acceptable carrier; and

wherein the patients in need thereof are administered by means of said pharmaceutical composition per day 250 to 500 μg vitamin B12, 0.5 to 1.5 vitamin B6 and 50 to 150 mg iron.

Clinical studies carried out by the present inventors showed that patients which have been subjected to gastric bypass surgery need to take exceptionally high amounts of vitamin B12 and iron to meet the desired levels of these nutrients. Clinical studies have further shown that in order to avoid hypervitaminosis of vitamin B6 it is important to provide less vitamin B6 than the amount which is recommended for healthy persons.

The pharmaceutical composition according to the present invention is preferably taken at least once every day as a single unit dose to meet said desired levels. It has further been found that it is advantageous to administer the iron in the absence of calcium, as calcium may interact with any iron available.

Clinical studies have further shown that treatment with common multivitamin compositions was not sufficient to reach a desired iron and vitamin B12 level in said patients. It has further been found that common multivitamin compositions comprise too much vitamin B6. With the pharmaceutical composition according to the present invention it has now for the first time been made possible to avoid iron and vitamin B12 deficiencies in patients which have been subjected to gastric bypass surgery. Although, it is theoretically possible to achieve with common multivitamin compositions the recommended vitamin B12 intake, the intake of several multivitamin tablets a day will lead to hypervitaminosis of other vitamins present in said common multivitamin compositions, such as hypervitaminosis of vitamin A, K or B6.

A second aspect of the present invention relates to a therapeutic combination for use in the treatment or prevention of vitamin and mineral deficiencies in patients which have been subjected to gastric bypass-surgery, wherein the combination comprises:

(a) a first unit dose comprising:

- 250 to 500 μg of vitamin B12;
- 0.5 to 1.5 mg of vitamin B6;
- 50 to 150 mg of iron;
- less than 0.1 mg calcium;
- less than 0.1 μg vitamin K and

(b) a second unit dose comprising

- 450 to 3000 mg calcium;
- 5 to 15 μg vitamin D
- 50 to 250 μg vitamin K.

The therapeutic combination according the present invention comprises a first unit dose which comprises remarkably high amounts of vitamin B12 and iron. As explained above, patients which have been subjected to a gastric bypass procedure, in particular a RYGB most often suffer from vitamin B12 and iron deficiencies. In order to meet the desired levels of these nutrients the patients are administered the first unit dose comprising the remarkably high amounts of vitamin B12 and iron. Furthermore, in order to avoid hypervitaminosis of vitamin B6 it is preferred to adminster to said patients a relatively low dose of vitamin B6.

Due the gastric bypass surgery deficiencies with respect to other nutrients are also commonly observed, in order to also balance these deficiencies these nutrients should also be provided. However, in order to avoid interaction between the vitamins and minerals of the first unit dose, these other nutrients are provided in a separate unit dose.

A fourth aspect of the present invention relates to a pharmaceutical composition as such, wherein a unit dose of said composition comprises:

- 250 to 500 μg of vitamin B12
- 0.5 to 1.5 mg of vitamin B6;
- 50 to 150 mg iron;
- less than 0.1 mg calcium
- less than 0.1 μg vitamin K
- 15 to 30 mg zinc;
- 400 to 800 μg folic acid;
- less than 1 mg, preferably less than 0.1 mg magnesium; and

a pharmaceutically acceptable excipient.

DEFINITIONS

The term *'pharmaceutical compositions'* as used herein has its conventional meaning and refers to a composition which is pharmaceutically acceptable.

The term *'pharmaceutically acceptable'* as used herein has its conventional meaning and refers to compounds, material, compositions and/or dosage forms, which are, within the scope of sound medical judgment suitable for contact with the tissues of mammals, especially humans, without excessive toxicity, irritation, allergic response and other problem complications commensurate with a reasonable benefit/risk ratio.

The term *'excipient'* as used herein has its conventional meaning and refers to a pharmaceutically acceptable ingredient, which is commonly used in the pharmaceutical technology for preparing a granulate, solid or liquid oral dosage formulation.

The term *'treatment'* as used herein has its conventional meaning and refers to curative, palliative and prophylactic treatment.

The term *'unit dose'* has its conventional meaning and refers to a dosage form which has the capacity of being administered as such to a subject, preferably a human, to be effective, and which can be readily handled and packaged, remaining as a physically and chemically stable unit dose comprising the therapeutic agent. Typical examples of unit doses are tablets and capsules.

The term *'fixed dose combination'* as used herein has its conventional meaning and refers to a combination of defined doses of two or more mineral or vitamins presented in a single unit dose (e.g. a tablet or a capsule) and administered as such.

DETAILED DESCRIPTION OF THE INVENTION

Besides the great benefits of bariatric surgery, there are also disadvantages for the patients such as hampered food intake, dumping, defecation problems and nutrient deficiencies resulting from malabsorption. Common deficiencies, found in patients which have been subjected to a gastric bypass procedure include iron and vitamin B12. Anemia is therefore very common in this group of patients. The deficiencies that occur after surgery are mostly the result of an inadequate intake and malabsorption of essential micronutrients.

The reduced absorption of micronutrients from the intestine occurs primarily in the interventions with a malabsorptive component and is largely the result of skipping the stomach, the duodenum and the proximal portion of the jejunum. Because the food after a RYGB no longer comes into contact with gastric acid before it reaches the intestines, certain nutrients are not converted to a form that can be absorbed. In addition, the duodenum and proximal jejunum are the most important absorption locations for many micronutrients.

In an attempt to balance the iron and vitamin B12 deficiencies in patients which have been subjected to a gastric bypass, these patients are prescribed standard multivitamin supplements, which supplements generally comprise 100% of the recommended daily allowance (RDA) for iron, vitamin B12 and other vitamins. However, in the clinical study carried out by the present inventors it has now for the first time been shown that the administration of such standard multivitamin preparations did not result in solving the iron and vitamin B12 deficiencies commonly observed in these types of patients. Furthermore, clinical studies also showed that the amount of vitamin B6 provided by means of these standard multivitamin supplements is too high for this patient group and leads of hypervitaminosis. The pharmaceutical composition according to the present invention aims to solve these problems.

A first aspect of the present invention relates to a pharmaceutical composition for use in the treatment or prevention of vitamin and mineral deficiencies in patients which have been subjected to gastric bypass-surgery comprising:

-    vitamin B12 or a source thereof;
-    vitamin B6 or a source thereof;
-    iron or a source thereof;
-    a pharmaceutically acceptable carrier; and

wherein the patients in need thereof are administered per day 250 to 500 μg vitamin B12, 0.5 to 1.5 mg vitamin B6 and 50 to 150 mg iron.

The inventors of the present invention remarkably found that in order to avoid iron and vitamin B12 deficiencies in these types of patients, extremely high doses of said nutrients need to be administered. The administration of standard multivitamin preparations, which generally comprise 100% of the RDA of said nutrients resulted in an average decline of vitamin B12 and ferretin in said patients. The administration of the pharmaceutical composition according to the present invention, comprising about 10.000 to 15.000% of the RDA for vitamin B 12 and 350 to 500% of the RDA for iron resulted in a marked increase of vitamin B12 and a steady ferretin level within said patients.

This is surprising for reasons that it was generally assumed in the scientific literature that the addition of higher amounts of vitamin B12 and iron would not be helpful due to the fact that after a gastric bypass operation (in particular after a RYGB) the release of intrinsic factor is significantly reduced, which significantly compromises vitamin B12 and iron absorption. Furthermore, also due to the reduced secretion of hydrochloric acid, pepsin and pancreatic enzymes it was assumed that increasing the amounts of vitamin B12 and iron would not have a beneficial effect. Thus, contrary to what was expected in the art, the present inventors found that extremely high doses of said nutrients avoids iron and B12 deficiencies in patients which have been subjected to a gastric bypass procedure, in particular a RYGB.

Although, it is theoretically possible to achieve with common multivitamin compositions the recommended vitamin B12 intake, the intake of several multivitamin tablets a day will lead to hypervitaminosis of other vitamins present in said common multivitamin compositions, such as hypervitaminosis of vitamin A, K or B6.

In this regard it is further noted that the administration of standard multivitamin preparations comprising 100% of the RDA for vitamin B6 already resulted in hypervitaminosis of said vitamin in patients which have been subjected to a gastric bypass procedure.

Depending on the gender and age of the patient, the amount of iron provided to said patients may vary. Men and post-menopausal women are preferably provided 50 to 80 mg of iron per day, whereas pre-menopausal women are preferably provided with 100 mg or more per day.

In a preferred embodiment of the present invention the pharmaceutical composition comprises a unit dose, which dose comprises the complete daily dosage of vitamins and minerals needed for patients which have been subjected to a gastric bypass procedure. Such a unit dose is preferably formulated as a tablet or capsule and comprises:

- 250 to 500 $\mu$g of vitamin B12;
- 0.5 to 1.5 mg of vitamin B6;
- 50 to 150 mg iron;
- less than 0.1 mg calcium;
- less than 0.1 $\mu$g vitamin K;

and a pharmaceutically acceptable excipient.

In order to avoid interactions between vitamin B12, iron and calcium the content of the latter is preferably kept low. Furthermore, it has also been found that it is important to keep the amount of vitamin K in said unit dose relatively low. Vitamin K is a known coagulant and may have a negative effect on anti-coagulants provided to said patients after they have been operated. Hence, from a safety perspective the amount of vitamin K in the composition according to the present invention is preferably less than 0.1 $\mu$g.

In a preferred embodiment of the present invention a unit dose of said composition comprises further:

- 15 to 30 mg zinc;
- 400 to 800 $\mu$g folic acid;
- less than1 mg, preferably less than 0.1 mg magnesium.

Although patients having been subjected to a gastric bypass operation often lack from a vitamin B12 and iron deficiency, they are also at risk for developing other deficiencies. In order to prevent this, the pharmaceutical composition according to the present invention and in particular a unit dose thereof also comprises the above mentioned vitamins and minerals. With respect to the use of zinc it is noted that additional zinc is necessary to avoid loss of hair after the surgery has been carried out.

The pharmaceutical composition according to the present invention is preferably formulated as a fixed dose combination. More preferably, the fixed dose combination is a solid dosage form, such as a capsule, tablet or powder which are relatively easy to administer.

Besides the above mentioned minerals and vitamins, the pharmaceutical composition according to the present invention also comprises a pharmaceutically acceptable excipient. Such an excipient may be chosen from ingredients which are commonly used in the pharmaceutical technology for preparing granulate, solid or liquid oral dosage formulations.

Examples of categories of excipients include, but are not limited to, binders, disintegrants, lubricants, glidants, fillers and diluents. One of ordinary skill in the art may select one or more of the aforementioned excipients with respect to the particular desired properties of the granulate and/or solid oral dosage form by routine experimentation and without any undue burden. The amount of each excipient used may vary within ranges conventional in the art. The following references which are all hereby incorporated by reference disclose techniques and excipients used to formulate oral dosage forms. See "The Handbook of Pharmaceutical Excipients", 4th edition, Rowe et al., Eds., American Pharmaceuticals Association (2003); and "Remington: The Science and Practice of Pharmacy", 20th edition, Gennaro, Ed., Lippincott Williams & Wilkins (2000).

A second aspect of the present invention relates to a therapeutic combination for use in the treatment or prevention of vitamin and mineral deficiencies in patients which have been subjected to gastric bypass-surgery, wherein the composition comprises:

(a) a first unit dose comprising:

- 250 to 500 μg of vitamin B12;
- 0.5 to 1.5 mg of vitamin B6
- 50 to 150 mg of iron;
- less than 0.1 mg calcium;
- less than 0.1 μg vitamin K and

(b) a second unit dose comprising

- 450 to 3000 mg calcium;
- 5 to 15 μg vitamin D; and optional
- 50 to 250 μg vitamin K.

The first unit dose of the therapeutic combination according to the present invention comprises the unit dose as already described above. In addition to said first unit dose the therapeutic combination according to the present invention also comprises a second unit dose, which dose comprises at least calcium, vitamin D and optionally vitamin K. However, in order to avoid interactions between iron on the one hand and the other nutrients on the other hand, a combination of two unit doses is provided.

Furthermore, with respect to vitamin K, it is noted that a small amount is needed for the body to be able to take up calcium. However, too much vitamin K may impair the function of anti-coagulants provided after the gastric bypass procedure. Hence, said second unit dose may also comprise vitamin K after the patient has recovered from the gastric-bypass procedure and the administration of anti-coagulants has been stopped.

The provision of extra vitamin D, calcium and optionally vitamin K is of importance to patients which have been subjected to a gastric bypass procedure because after the operation the pressure on weight-bearing bones is reduced by the rapid weight loss. This reduction causes lower stimulation of the osteoclasts, which in turn causes osteoblasts to produce less bone. This first phase lasts about three months. A second phase of bone resorption takes place six months postoperatively. This phase is due to a lack of intake of vitamin D and an insufficient absorption of calcium. The response of the body to the shortage of calcium is to absorb calcium from the skeleton reducing its strength. After one year, the bone loss in all the bones, especially in weight-bearing bones like the pelvis and lower back, has become significantly large at 7.8% to 10.5%. In the long term this is very detrimental to the patient as they develop a significantly greater risk of fractures. Hence, additional vitamin D and calcium is needed for this group of patients.

The combination according to the present invention is preferably administered at least once per day. In special cases, the combination may be administered more times a day, although it is assumed that this is only beneficial for a limited amount of time, e.g. one to three months.

In order to also avoid interactions of the different vitamins and minerals in the body it is preferred to administer the second unit dose to a patient in need thereof at least one hour, preferably at least two hours and more preferably at least three hours after or before administering the first unit dose to said patient.

Preferably, the first unit dose comprises further:

- 15 to 30 mg zinc;
- 400 to 800 μg folic acid;
- less than1 mg, preferably less than 0.1 mg magnesium.

The first unit dose and/or the second unit dose is preferably formulated as a fixed dose combination, preferably an oral fixed dose combination, most preferably a tablet, capsule or powder.

Besides the vitamins and minerals the unit doses of the therapeutic combination according to the present invention also comprises a pharmaceutically acceptable excipient. Suitable excipient for use in the first and second unit doses include, but are not limited to, binders, disintegrants, lubricants, glidants, fillers and diluents. One of ordinary skill in the art may select one or more of the aforementioned excipients with respect to the particular desired properties of the granulate and/or solid oral dosage form by routine experimentation and without any undue burden.

The amount of each excipient used may vary within ranges conventional in the art. The following references which are all hereby incorporated by reference disclose techniques and excipients used to formulate oral dosage forms. See "The Handbook of Pharmaceutical Excipients", 4th edition, Rowe et al., Eds., American Pharmaceuticals Association (2003); and "Remington: The Science and Practice of Pharmacy", 20th edition, Gennaro, Ed., Lippincott Williams & Wilkins (2000).

A last aspect of the present invention relates to a pharmaceutical composition as such comprising:

- 250 to 500 μg of vitamin B12
- 0.5 to 1.5 mg of vitamin B6;
- 50 to 150 mg iron;
- less than 0.1 mg calcium
- less than 0.1 μg vitamin K;
- 15 to 30 mg zinc;
- 400 to 800 μg folic acid;
- less than1 mg, preferably less than 0.1 mg magnesium; and

a pharmaceutically acceptable excipient.

Besides the vitamins and minerals, the pharmaceutical composition also comprises a pharmaceutically acceptable excipient. Suitable excipient for use in the first and second unit doses include, but are not limited to, binders, disintegrants, lubricants, glidants, fillers and diluents. One of ordinary skill in the art may select one or more of the aforementioned excipients with respect to the particular desired properties of the granulate and/or solid oral dosage form by routine experimentation and without any undue burden.

The amount of each excipient used may vary within ranges conventional in the art. The following references which are all hereby incorporated by reference disclose techniques and excipients used to formulate oral dosage forms. See "The Handbook of Pharmaceutical Excipients", 4th edition, Rowe et al., Eds., American Pharmaceuticals Association (2003); and "Remington: The Science and Practice of Pharmacy", 20th edition, Gennaro, Ed., Lippincott Williams & Wilkins (2000).

The pharmaceutical composition according the present invention is preferably formulated as a unit dose, preferably as an oral dosage form such as a tablet or capsule.

The present invention will be illustrated further by means of the following nonlimiting example.

**EXAMPLE**

**Double blind, randomized study comparing a pharmaceutical composition (WLS forte) according to the present invention with a standard multivitamin supplement (sMVS)**

A double-blind, randomized, 12-month study was conducted comparing WLS forte with a standard multivitamin supplement (sMVS) containing approximately 100% of recommended daily allowance (RDA) for iron, vitamin B12 and folic acid. WLS Forte contains vitamin B12 at a level 14000% RDA, iron at 500% RDA, and folic acid at 300% RDA per capsule.

Patients

Between June 2011 and March 2012 a total of 150 patients who were scheduled for a primary laparoscopic RYGB (LRYGB) were randomized for postoperative multivitamin supplementation for a duration of twelve months. All patients met the criteria for bariatric surgery according to NIH Consensus Development Conference Panel for bariatric surgery. Patients were randomized to receive either standard multivitamin supplements (sMVS, FitForMe, Rotterdam, The Netherlands) or RYGB specific multivitamin supplements (WLS Forte, FitForMe, Rotterdam, The Netherlands). A computer-generated variable block schedule was used for randomization. Patients, surgeons and researchers were blinded for the supplements. Both supplements were similar for color, size, and packaging, and both were dosed as one capsule daily. The composition of both supplements is shown in Table 1. The sMVS served as control and contained the compounds of interest in a dose equivalent to the recommended daily allowance (RDA), whereas WLS Forte contained much higher doses, in particular, of iron (5 times RDA) and vitamin B12 (140 times RDA).

Surgical procedure

All procedures were performed by one of 3 bariatric surgeons all beyond their learning curve (> 750 procedures each). They performed an antecolic antegastric LRYGB, with a proximal gastric pouch of 30 ml, a biliopancreatic limb (BPL) of 50 cm and a Roux limb of 150 cm. All patients received low-molecular heparin (Nadroparin 5700IU daily) for 6 weeks and proton-pump inhibitor (Omeprazol 40mg daily) for 6 months, as a part of our standard postoperative protocol.

Follow-up and outcome

All patients followed a strict postoperative schedule consisting of 17 visits in the first year, and on each visit patients were encouraged to keep taking their supplements. Standard laboratory blood test were performed at baseline, 6 and 12 months. This included a complete blood count (CBC, (normal range (NR) hemoglobin: females: 7.4 - 9.9 mmol/L, males: 8.4 - 10.8 mmol/L), mean cell volume (MCV, [NR: 80 - 100 fL]), creatinine [NR: 45 - 90 $\mu$mol/L], sodium [NR: 135 - 145 mmol/l], potassium [NR: 3.5 - 4.7 mmol/l], calcium [NR: 2.10 - 2.55 mmol/l], phosphate [NR: 0.87 - 1.45 mmol/l], magnesium [NR: 0.71 - 0.93 mmol/l], zinc [NR 9.2 - 18.4 $\mu$mol/L], albumin [35 - 50 g/l], fasting glucose (FG, [NR: 4.0 - 5.6 mmol/l]), Hb1Ac [NR: 20 - 42 mmol/mol], total cholesterol [NR <6.50 mmol/l], HDL-cholesterol [NR >1.10 mmol/l], LDL-cholesterol [NR: 3.50 - 4.50 mmol/l], and triglycerides [NR: 0.8 - 2.0 mmol/l]), iron [NR: 9.0 - 31.0 $\mu$mol/l], total-iron-binding-capacity (TIBC, [NR: 45.0 - 81.0 $\mu$mol/L]), ferritin [NR 20 -200 $\mu$g/L], folic acid [NR: 9.0 - 36.0 nmol/l], vitamin B12 [NR: 150 - 640 $\mu$mol/l], 25-hydroxyvitamin D (25-OHD, [NR: >50 nmol/L]), parathyroid hormone (PTH, [NR: 1.3 - 6.8 pmol/l]), vitamin B1 [NR: 95 - 175 nmol/L], vitamin B6 [NR: 25 - 100 nmol/L].

Primary outcome variables were the percentage of iron and vitamin B12 deficiencies developed during the 12 month after LRYGB. Iron Deficiency (ID), was defined as a serum ferritin < 20 $\mu$g/L and vitamin B12 deficiency if the level was <150 pmol/L. Vitamin D deficiency was diagnosed if 25-OHD < 50 nmol/L, hypocalcemia if serum calcium <2.1 mmol/L), and zinc deficiency of the serum level was <9.2 $\mu$mol/L. Calcium data are shown as calcium levels corrected for albumin 130 (Cacorr), according to the following equation:

$$Ca_{corr} = Total\ Calcium - (0.025\ x\ albumin) + 1$$

Statistical analysis

Sample size calculation was performed by the epidemiologist of the Research Department of Rijnstate Hospital in Arnhem (NL) using Openepi.com. Sample size calculation was based on the number of patients developing ID. To detect a 25% reduction of ID 12 months after surgery, with a confidence interval 95% and a power of 90%, a minimum of 56 patients per group were needed. Taking into account a 10% drop-out and 15% deficiency of iron at 6 months which will be supplemented at that time, it was decided to include 75 patients per 160 treatment group.

All data were analyzed using IBM® SPSS® Statistics 20 for Windows. Data were expressed in mean ($\pm$ standard deviation), unless otherwise specified. Difference between groups were calculated using student-t test for continuous data and chi-square test for ordinal/ nominal data. A P-value < 0.05 was considered statistically significant.

**Results**

Two patients were excluded after randomization: one patient in the WLS Forte group because he underwent a sleeve gastrectomy instead of a LRYGB because of multiple adhesions during surgery, and one patient in the sMVS group because he cancelled the scheduled operation procedure. In total 148 patient (74 in each group) underwent a LRYGB and were included for analysis. Both groups were similar with respect to age, sex, weight, body mass index (BMI) and preoperative deficiencies. However, dyslipidaemia was twice as frequent in the WLS Forte group as compared to the sMVS group (P = 0.04).

Weight loss

The degree of weight loss over 12 months was similar in both groups. Weight dropped to 90.6$\pm$17.4 kg compared 93.8$\pm$16.9 kg in sMVS versus WLS Forte (p=0.24), respectively. Percentage Excess Weight Loss (%EWL), defined as weight loss divided by excess weight based on ideal body weight at BMI 25 kg/m2, were after 12 months 72.5$\pm$20.9 180 kg/m2 versus 72.1$\pm$23.2 kg/m2 for MVS and WLS Forte (p=0.92), respectively.

Iron

The total number of patients developing ferritin deficiency during follow-up were 8 (10.7%) patients in sMVS and 1 (1.3%) patients in WLS Forte (p=0.03). In total 55 (37.2%) patients, 28 (37.8%) in sMVS and 27 (36.5%) in WLS Forte group, received additional iron medication at any time during the 12 month follow-up. Results after exclusion of these patients are shown in Table 4. Mean serum ferritin decreased by 18.4$\pm$61.8 ug /L in the sMVS group, but remained stable in the WLS Forte group (p = 0.08).

V itamin B12

Mean vitamin B12 serum levels decreased by -38.9±141.3 pmol/L in the sMVS group and increased by 44.1±138.8 pmol/L in the WLS Forte group (<0.001) after 12 months, and as a result mean vitamin B12 blood serum levels at 6 months and 12 months were significant higher with WLS Forte compared to sMVS (p<0.05). After 12 months, vitamin B12 deficiency had developed in 5 (7.9%) patients receiving sMVS versus 1 (1.6%) in WLS Forte group (p=0.207).

Table 1: Dosages of supplement ingredients

| Ingredients | Standard MVS | | WLS Forte | |
|---|---|---|---|---|
| | *Dosage* | *RDA (%)* | *Dosage* | *RDA (%)* |
| Biotin (μg) | 25.00 | 50.0 | 600.0 | 1200.0 |
| Calcium (mg) | 91.43 | 110.4 | 0.0 | 0.0 |
| Chloride (mg) | 0.14 | 0.0 | 0.54 | 0.1 |
| Chrome (μg) | 40.00 | 100.0 | 160.00 | 400.0 |
| Copper (μg) | 999.60 | 100.0 | 3000.20 | 300.0 |
| Folic acid (μg) | 200.00 | 100.0 | 600.00 | 300.0 |
| Iodine (μg) | 153.70 | 102.5 | 225.04 | 150.0 |
| Iron (mg) | 14.00 | 100.0 | 70.00 | 500.0 |
| Manganese (mg) | 2.00 | 100.1 | 3.00 | 150.0 |
| Magnesium (mg) | 30.00 | 8.0 | 0.0 | 0.0 |
| Molybdenum (μg) | 50.00 | 100.0 | 112.40 | 224.8 |
| Selenium (μg) | 55.00 | 100.0 | 105.00 | 190.9 |
| Vitamin A (μg) | 599.70 | 75.0 | 600.00 | 75.0 |
| Vitamin B1 (mg) | 1.10 | 99.7 | 2.75 | 249.7 |
| Vitamin B2 (mg) | 1.40 | 100.0 | 3.50 | 250.0 |
| Vitamin B3 (mg) | 16.0 | 100.0 | 32.00 | 200.0 |
| Vitamin B5 (mg) | 6.00 | 100.0 | 18.00 | 300.1 |
| Vitamin B6 (mg) | 1.40 | 100.2 | 0.98 | 70.0 |
| Vitamin B12 (μg) | 12.50 | 100.0 | 350.00 | 14000.0 |
| Vitamin C (mg) | 80.00 | 100.0 | 120.00 | 150.0 |
| Vitamin D (μg) | 4.00 | 80.0 | 12.50 | 250.0 |
| Vitamin E (mg) | 10.00 | 83.4 | 24.00 | 200.0 |
| Vitamin K1 (μg) | 25.00 | 33.3 | < 0.01 | 0.0 |
| Zinc (mg) | 10.00 | 100.0 | 22.50 | 225.0 |
| *RDA: European Recommended Dietary Allowance* | | | | |

Table 2: Laboratory blood tests of hemoglobin metabolism excluding patients with additional iron and /or vitamin B 12 medication

| Serum levels | Standard MVS | WLS Forte | p-value |
|---|---|---|---|
| *Hemoglobin (mmol/L)* | | | |
| Baseline | 8.5 ± 0.6 | 8.6 ± 0.7 | 0.38 |

(continued)

| Serum levels | Standard MVS | WLS Forte | p-value |
|---|---|---|---|
| 6 months | 8.6 ± 0.7 | 8.4 ± 0.7 | 0.25 |
| 12 months | 8.5 ± 0.8 | 8.5 ± 0.6 | 1.00 |
| Δ 0 - 12 months | 0.029 ± 0.7 | (± 0.5 | 0.64 |
| *Ferritin (μg/L)* | | | |
| Baseline | 103.0 ± 86.1 | 102.1 ± 71.0 | 0.95 |
| 6 months | 84.5 ± 73.2 | 97.8 ± 66.7 | 0.29 |
| 12 months | 80.8 ± 71.0 | 108.3 ± 83.1 | 0.05 |
| Δ 0 - 12 months | -18.4 ± 61.8 | 4.9 ± 81.3 | 0.08 |
| *Vitamin B12 (pmol/L)* | | | |
| Baseline | 305.5 ± 107.1 | 302.8 ± 100.9 | 0.88 |
| 6 months | 249.9 ± 85.6 | 351.3 ± 135.6 | <0.001 |
| 12 months | 267.2 ± 100.1 | 349.8 ± 122.1 | <0.001 |
| Δ 0 - 12 months | (± 141.3 | 44.1 ± 138.8 | 0.002 |
| *Numbers are mean (±SD), MVS: Multivitamin Supplement* | | | |

## Claims

1. Pharmaceutical composition for use in the treatment or prevention of vitamin and mineral deficiencies in patients which have been subjected to gastric bypass-surgery comprising:

   - vitamin B 12 or a source thereof;
   - vitamin B6 or a source thereof;
   - iron or a source thereof;
   - a pharmaceutically acceptable carrier; and

   wherein the patients in need thereof are administered by means of said composition per day 250 to 500 μg vitamin B12, 0.5 to 1.5 mg vitamin B6 and 50 to 150 mg iron.

2. Pharmaceutical composition for use according to claim 1, wherein a unit dose of the composition comprises:

   - 250 to 500 μg of vitamin B12;
   - 0.5 to 1.5 mg of vitamin B6;
   - 50 to 150 mg iron;
   - less than 0.1 mg calcium;
   - less than 0.1 μg vitamin K; and

   a pharmaceutically acceptable excipient.

3. Pharmaceutical composition according to claim 1 or 2, for use in patients which have been subjected to a Roux-en-Y Gastric Bypass (RYGB).

4. Pharmaceutical composition for use according to any of the previous claims, wherein the composition is administered at least once daily to patients in need thereof.

5. Pharmaceutical composition for use according to any of the previous claims, wherein a unit dose of said composition comprises further:

- 15 to 30 mg zinc;
- 400 to 800 μg folic acid;
- less than 1 mg, preferably less than 0.1 mg magnesium.

6. Pharmaceutical composition for use according to any of the previous claims, wherein the composition is formulated as a fixed dose combination, preferably as an oral fixed dose combination.

7. Pharmaceutical composition for use according to the previous claim, wherein the oral fixed dose combination is a solid dosage form, such as a capsule, tablet or powder.

8. Therapeutic combination for use in the treatment or prevention of vitamin and mineral deficiencies in patients which have been subjected to gastric bypass-surgery, wherein the composition comprises:

    (a) a first unit dose comprising:

       - 250 to 500 μg of vitamin B12;
       - 0.5 to 1.5 mg of vitamin B6;
       - 50 to 150 mg of iron;
       - less than 0.1 mg calcium;
       - less than 0.1 μg vitamin K; and

    (b) a second unit dose comprising

       - 450 to 3000 mg calcium;
       - 5 to 15 μg vitamin D.
       - 50 to 250 μg vitamin K.

9. Therapeutic combination according to claim 8, for use in patients which have been subjected to a Roux-en-Y Gastric Bypass (RYGB).

10. Therapeutic combination for use according to claim 8 or 9, wherein the combination is administered at least once daily to patients in need thereof.

11. Therapeutic combination for use according to any of the claims 8-10, wherein the second unit dose is administered to a patient in need thereof at least one hour, preferably at least two hours and more preferably at least three hours after or before administering the first unit dose to said patient.

12. Therapeutic combination for use according to any of the claims 8-11, wherein the first unit dose comprises further:

       - 15 to 30 mg zinc;
       - 400 to 800 μg folic acid;
       - less than 1 mg, preferably less than 0.1 mg magnesium.

13. Therapeutic combination according to any of the claims 8-12, wherein the first unit dose and/or the second unit dose is a fixed dose combination, preferably an oral fixed dose combination, most preferably a tablet, capsule or powder.

14. Pharmaceutical composition comprising:

       - 250 to 500 μg of vitamin B12;
       - 0.5 to 1.5 mg of vitamin B6
       - 50 to 150 mg iron;
       - less than 0.1 mg calcium;
       - less than less than 0.1 μg vitamin K;
       - 15 to 30 mg zinc;
       - 400 to 800 μg folic acid;
       - less than 1 mg, preferably less than 0.1 mg magnesium; and

    a pharmaceutically acceptable excipient.

**Patentansprüche**

1. Pharmazeutische Zusammensetzung zur Verwendung bei der Behandlung oder Prävention von Vitaminmangel und Mineralienmangel bei Patienten, die einer gastrischen Umgehungschirurgie unterzogen wurden, umfassend:

   - Vitamin B 12 oder eine Quelle davon;
   - Vitamin B6 oder eine Quelle davon;
   - Eisen oder eine Quelle davon;
   - eine pharmazeutisch akzeptable Trägersubstanz; und

   wobei den Patienten, die diese dringend brauchen, mittels der Zusammensetzung pro Tag 250 bis 500 $\mu$g Vitamin B12, 0,5 bis 1,5 mg Vitamin B6 und 50 bis 150 mg Eisen verabreicht werden.

2. Pharmazeutische Zusammensetzung zur Verwendung nach Anspruch 1, wobei eine Einheitsdosis der Zusammensetzung enthält:

   - 250 bis 500 $\mu$g Vitamin B12;
   - 0,5 bis 1,5 mg Vitamin B6;
   - 50 bis 150 mg Eisen;
   - weniger als 0,1 mg Calcium;
   - weniger als 0,1 $\mu$g Vitamin K; und

   einen pharmazeutisch akzeptablen Trägerstoff.

3. Pharmazeutische Zusammensetzung nach Anspruch 1 oder 2, zur Verwendung bei Patienten, die einem Magenbypass Roux-en-Y (RYGB) unterzogen wurden.

4. Pharmazeutische Zusammensetzung zur Verwendung nach einem der vorhergehenden Ansprüche, wobei die Zusammensetzung den Patienten, die sie dringend brauchen, mindestens einmal täglich verabreicht wird.

5. Pharmazeutische Zusammensetzung zur Verwendung nach einem der vorhergehenden Ansprüche, wobei eine Einheitsdosis der Zusammensetzung des Weiteren enthält:

   - 15 bis 30 mg Zink;
   - 400 bis 800 $\mu$g Folsäure;
   - weniger als 1 mg, vorzugsweise weniger als 0,1 mg, Magnesium.

6. Pharmazeutische Zusammensetzung zur Verwendung nach einem der vorhergehenden Ansprüche, wobei die Zusammensetzung als eine festgelegte Dosiskombination, vorzugsweise als eine orale festgelegte Dosiskombination angesetzt ist.

7. Pharmazeutische Zusammensetzung zur Verwendung nach dem vorhergehenden Anspruch, wobei die orale festgelegte Dosiskombination eine feste Dosierungsform wie etwa eine Kapsel, Tablette oder Pulver ist.

8. Therapeutische Kombination zur Verwendung bei der Behandlung oder Prävention von Vitaminmangel und Mineralienmangel bei Patienten, die einer gastrischen Umgehungschirurgie unterzogen wurden, wobei die Zusammensetzung enthält:

   (a) eine erste Einheitsdosis, enthaltend:

   - 250 bis 500 $\mu$g Vitamin B12;
   - 0,5 bis 1,5 mg Vitamin B6;
   - 50 bis 150 mg Eisen;
   - weniger als 0,1 mg Calcium;
   - weniger als 0,1 $\mu$g Vitamin K; und

   (b) eine zweite Einheitsdosis, enthaltend:

- 450 bis 3000 mg Calcium;
- 5 bis 15 μg Vitamin D;
- 50 bis 250 μg Vitamin K.

9. Therapeutische Kombination nach Anspruch 8 zur Verwendung in Patienten, die einem Magenbypass Roux-en-Y (RYGB) unterzogen wurden.

10. Therapeutische Kombination zur Verwendung nach Anspruch 8 oder 9, wobei die Kombination mindestens einmal täglich an Patienten verabreicht wird, die sie dringend brauchen.

11. Therapeutische Kombination zur Verwendung nach einem der Ansprüche 8 bis 10, wobei die zweite Einheitsdosis einem Patienten verabreicht wird, der sie dringend braucht, mindestens eine Stunde, vorzugsweise mindestens zwei Stunden und besser mindestens drei Stunden nach oder vor Verabreichung der ersten Einheitsdosis an den Patienten.

12. Therapeutische Kombination zur Verwendung nach einem der Ansprüche 8 bis 11, wobei die erste Einheitsdosis außerdem enthält:

- 15 bis 30 mg Zink;
- 400 bis 800 μg Folsäure;
- weniger als 1 mg, vorzugsweise weniger als 0,1 mg, Magnesium.

13. Therapeutische Kombination nach einem der Ansprüche 8 bis 12, wobei die erste Einheitsdosis und/oder die zweite Einheitsdosis eine festgelegte Dosiskombination, vorzugsweise eine orale festgelegte Dosiskombination, am besten eine Tablette, Kapsel oder Pulver ist.

14. Pharmazeutische Zusammensetzung, enthaltend:

- 250 bis 500 μg Vitamin B12;
- 0,5 bis 1,5 mg Vitamin B6;
- 50 bis 150 mg Eisen;
- weniger als 0,1 mg Calcium;
- weniger als 0,1 μg Vitamin K;
- 15 bis 30 mg Zink;
- 400 bis 800 μg Folsäure;
- weniger als 1 mg, vorzugsweise weniger als 0,1 mg, Magnesium; und

einen pharmazeutisch akzeptablen Trägerstoff.

**Revendications**

1. Composition pharmaceutique à utiliser dans le traitement ou la prévention de déficiences de vitamines et de minéraux chez des patients qui ont été soumis à une chirurgie de dérivation gastrique comprenant :

de la vitamine B12 ou une source de celle-ci ;
de la vitamine B6 ou une source de celle-ci ;
du fer ou une source de celui-ci ;
un support acceptable pharmaceutiquement ; et

dans laquelle on administre quotidiennement aux patients ayant besoin de celle-ci une composition de 250 à 500 μg de vitamine B12, de 0,5 à 1,5 mg de vitamine B6 et de 50 à 150 mg de fer.

2. Composition pharmaceutique à utiliser conformément à la revendication 1, dans laquelle une dose unitaire de la composition comprend :

de 250 à 500 μg de vitamine B12 ;
de 0,5 à 1,5 mg de vitamine B6 ;

de 50 à 150 mg de fer ;
moins de 0,1 mg de calcium ;
moins de 0,1 $\mu$g de vitamine K ; et

un excipient acceptable pharmaceutiquement.

3. Composition pharmaceutique selon la revendication 1 ou 2, à utiliser chez des patients qui ont été soumis à une dérivation gastrique de Roux-en-Y (RYGB).

4. Composition pharmaceutique à utiliser conformément à l'une quelconque des revendications précédentes, dans laquelle la composition est administrée au moins une fois par jour à des patients ayant besoin de celle-ci.

5. Composition pharmaceutique à utiliser conformément à l'une quelconque des revendications précédentes, dans laquelle une dose unitaire de la composition comprend de plus :

de 15 à 30 mg de zinc ;
de 400 à 800 $\mu$g d'acide folique ;
moins de 1 mg, de préférence moins de 0,1 mg de magnésium.

6. Composition pharmaceutique à utiliser conformément à l'une quelconque des revendications précédentes, dans laquelle la composition est formulée sous forme d'une combinaison à dose fixée, de préférence sous la forme d'une combinaison à dose fixée orale.

7. Composition pharmaceutique à utiliser conformément à la revendication précédente, dans laquelle la combinaison à dose fixée orale est une forme de dosage solide, telle qu'une capsule, un comprimé ou une poudre.

8. Combinaison thérapeutique à utiliser dans le traitement ou la prévention de déficiences en vitamines et minéraux chez des patients qui ont été soumis à une chirurgie de dérivation gastrique, dans laquelle la composition comprend :

(a) une première dose unitaire comprenant :

de 250 à 500 $\mu$g de vitamine B12 ;
de 0,5 à 1,5 mg de vitamine B6 ;
de 50 à 150 mg de fer ;
moins de 0,1 mg de calcium ;
moins de 0,1 $\mu$g de vitamine K ; et

(b) une seconde dose unitaire comprenant :

de 450 à 3000 mg de calcium ;
de 5 à 15 $\mu$g de vitamine D ;
de 50 à 250 $\mu$g de vitamine K.

9. Combinaison thérapeutique selon la revendication 8, pour utilisation chez des patients qui ont été soumis à une dérivation gastrique de Roux-en-Y (RYGB).

10. Combinaison thérapeutique à utiliser selon les revendications 8 ou 9, dans laquelle la combinaison est administrée au moins une fois par jour à des patients ayant besoin de celle-ci.

11. Combinaison thérapeutique à utiliser selon l'une quelconque des revendications 8 à 10, dans laquelle la seconde dose unitaire est administrée à un patient ayant besoin de celle-ci au moins une heure, de préférence au moins deux heures et de manière plus préférée au moins trois heures après ou avant d'administrer la première dose unitaire au patient.

12. Combinaison thérapeutique à utiliser conformément à l'une quelconque des revendications 8 à 11, dans laquelle la première dose unitaire comprend de plus :

de 15 à 30 mg de zinc ;

de 400 à 800 µg d'acide folique ;
moins de 1 mg, de préférence moins de 0,1 mg de magnésium.

13. Combinaison thérapeutique selon l'une quelconque des revendications 8 à 12, dans laquelle la première dose unitaire et/ou la seconde dose unitaire est une combinaison à dose fixée, de préférence une combinaison à dose fixée orale, de la manière la plus préférée un comprimé, une capsule ou une poudre.

14. Composition pharmaceutique comprenant :

de 250 à 500 µg de vitamine B12 ;
de 0,5 à 1,5 mg de vitamine B6 ;
de 50 à 150 mg de fer ;
moins de 0,1 mg de calcium ;
moins de 0,1 µg de vitamine K ;
de 15 à 30 mg de zinc ;
de 400 à 800 µg d'acide folique ;
moins de 1 mg, de préférence moins de 0,1 mg de magnésium ; et

un excipient acceptable pharmaceutiquement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

graphy">**Non-patent literature cited in the description**

- The Handbook of Pharmaceutical Excipients. American Pharmaceuticals Association, 2003

- Remington: The Science and Practice of Pharmacy. Lippincott Williams & Wilkins, 2000

**17**